(19) Europäisches Patentamt
European Patent Office
Office européen des brevets

(11) **EP 4 537 852 A1**

(12) **EUROPEAN PATENT APPLICATION**
published in accordance with Art. 153(4) EPC

(43) Date of publication:
**16.04.2025 Bulletin 2025/16**

(21) Application number: **23807904.0**

(22) Date of filing: **17.05.2023**

(51) International Patent Classification (IPC):
***A61K 47/64*** *(2017.01)* ***A61K 47/68*** *(2017.01)*
***A61K 31/437*** *(2006.01)* ***A61P 35/00*** *(2006.01)*

(52) Cooperative Patent Classification (CPC):
**A61K 31/437; A61K 47/64; A61K 47/68; A61P 35/00**

(86) International application number:
**PCT/KR2023/006685**

(87) International publication number:
**WO 2023/224385 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 KR 20220061077**

(71) Applicant: **Kmd Bio Co., Ltd.**
**Daejeon 34015 (KR)**

(72) Inventors:
- **KIM, Myung Hoon**
  **Seoul 08011 (KR)**
- **RHO, Jong Kook**
  **Daejeon 34120 (KR)**
- **BYUN, Seung Min**
  **Daejeon 34018 (KR)**
- **CHOI, Hyo Jin**
  **Sejong 30151 (KR)**
- **LEE, Gyeong Hee**
  **Daejeon 35201 (KR)**

(74) Representative: **Cooley (UK) LLP**
**22 Bishopsgate**
**London EC2N 4BQ (GB)**

Remarks:
The complete document including Reference Table(s) and the Sequence Listing(s) can be downloaded from the EPO website

(54) **HER2-GST-SN-38 COMPLEX, AND PHARMACEUTICAL COMPOSITION COMPRISING SAME FOR PREVENTING OR TREATING PROLIFERATIVE DISEASES**

(57) Provided are a fusion protein including a glutathione-S-transferase and a protein having binding ability to a target cell or a target protein, and a drug complex thereof, and use thereof as a pharmaceutical composition. The fusion protein according to an aspect and the drug complex including the same can sustain a prolonged residence time *in vivo,* and can be effectively delivered to target cells due to an improved ability to target the target cells, and thus can be effectively used as a targeted therapeutic agent.

FIG. 5

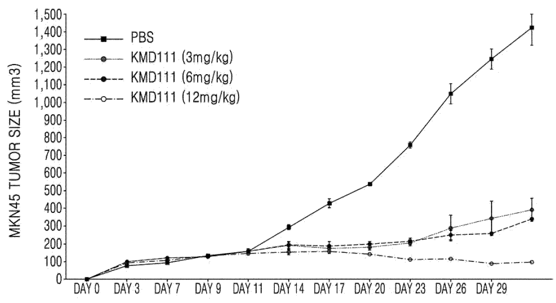


EP 4 537 852 A1

## Description

### Technical Field

The present invention relates to a fusion protein including glutathione-S-transferase and a protein having binding ability to a target cell or a target protein, and use of the fusion protein as a drug delivery carrier, a drug complex, and a pharmaceutical composition.

### Background Art

Nanoparticles have excellent biological distribution properties and can control the degree of drug release, and thus may be used in the fields of imaging devices, targeted therapeutics, etc. Typically, nanoparticles with a particle diameter of 200 nm may leak into blood vessels in the tumor periphery, and the leaked nanoparticles are known to continuously remain in the tumor tissue because of a low pressure due to a lack of the formation of lymphatic vessels around the tumor. Such a process is called an enhanced permeability and retention effect (EPR effect), through which permeability and retention of a drug delivery carrier may be improved.

Representative examples of nanoparticles that are commercially available today may include Abraxane and Doxil. However, there are limitations in that the nanoparticles show different vascular permeability for different tumors, and when the nanoparticles are intravenously administered, the nanoparticles may rapidly accumulate in reticuloendothelial organs such as the liver and spleen to avoid removal thereof by the mononuclear phagocyte system (MPS). In this regard, targeted therapeutic agents to which nanoparticles are applied may have reduced therapeutic effects, and may exhibit side effects due to toxicity of nanoparticles.

To overcome these advantages, a method of surrounding nanoparticles by poly(ethylene glycol)ylation (PEGylation) has been developed. When this method is used, there is an advantage in that the time for nanoparticles to circulate in the circulatory system *in vivo* may be prolonged, but conversely, the possibility of uptake by target cells may be reduced, and cells other than the target cells may be targeted through non-specific binding, which may also reduce therapeutic efficiency.

In using nanoparticles as a drug delivery carrier, strategies to induce structural changes in nanoparticles have been suggested to enhance the EPR effect along with targeting ability for drug delivery. Specifically, there have been continuous attempts to improve targeting ability of drug delivery carriers by inducing structural changes, such as coating the surface of nanoparticles with antibodies, proteins, or peptides capable of binding to receptors overexpressed in cancer cells. However, even through this method, the tumor-targeting efficiency is not effectively increased, and rather, a target ligand capable of more rapidly removing nanoparticles through an *in vivo* immune response by the MPS may be provided, resulting in a limitation in that a significant therapeutic effect is not exhibited in terms of overall tumor therapeutic effects.

Theoretically, when exposed to a physiological environment, the surface of nanoparticles is naturally surrounded by other biomolecules toward lowering the surface energy by combination of actions such as arrangement of water molecules according to the entropy, charge compensation of the particle surface, exposure of hydrophobic moieties, etc. Here, various biomolecules are non-specifically adsorbed onto the surface of nanoparticles, and this form is called a protein corona. The protein corona is surrounded by other molecules, changing its original molecular characteristics, and targeting ability to target cells or organs and various biological functions exhibited by nanoparticles may be blocked. In this regard, research is ongoing to control the protein corona for application of nanoparticles as a targeted therapeutic agent at a clinical level.

Therefore, in a process of preparing a nanoparticle-based targeted therapeutic agent, a method of controlling a protein corona after forming the protein corona on the surface of nanoparticles was developed. To this end, disclosed is a method of minimizing an interaction with serum proteins by modifying the surface of nanoparticles with zwitterionic, PEG, carbohydrate residues, etc., to avoid blocking of the targeting ability by the protein corona. Furthermore, the protein corona may be controlled through circulation of a desired protein in plasma by pre-coating nanoparticles with dysopsonic proteins. Accordingly, nanoparticles that are pre-coated with the protein corona may have increased stability at a colloidal state, and thus may not be removed by the MPS and may exhibit an effect of sustaining the circulation time of the nanoparticles in the blood. However, even through this method, the targeting ability to a target may be restricted, and since biochemical actions from biological interactions between the nanoparticles and the proteins used in the pre-coating are unknown, clinical application of the nanoparticles also has limitations.

### Disclosure

### Technical Problem

An aspect provides a drug complex including: a glutathione-S-transferase (GST) molecule; an antibody, an

affibody molecule, or a diabody molecule, each having binding ability to human epidermal growth factor receptor 2 (HER2); a linker that links the GST with the antibody, the affibody molecule, or the diabody molecule; and 7-ethyl-10-hydroxycamptothecin bound with the GST via a glutathione (GSH) molecule.

Another aspect provides a pharmaceutical composition for preventing or treating a proliferative disease, the pharmaceutical composition including: a GST molecule; an antibody, an affibody molecule, or a diabody molecule, each having binding ability to HER2; a linker that links the GST with the antibody, the affibody molecule, or the diabody molecule; and 7-ethyl-10-hydroxycamptothecin bound with the GST via a GSH molecule.

Another aspect provides a method of preventing or treating a proliferative disease, the method including administering an effective amount of a drug complex to an individual in need thereof, wherein the drug complex includes: a GST molecule; an antibody, an affibody molecule, or a diabody molecule, each having binding ability to HER2; a linker that links the GST with the antibody, the affibody molecule, or the diabody molecule; and 7-ethyl-10-hydroxycamptothecin bound with the GST via a GSH molecule.

Another aspect provides use of a drug complex for the manufacture of a pharmaceutical preparation for preventing or treating a proliferative disease, the drug complex including: a GST molecule; an antibody, affibody or diabody molecule having a binding ability to HER2; a linker that links the GST with the antibody, the affibody ability, or the diabody ability; and 7-ethyl-10-hydroxycamptothecin bound with the GST via a GSH molecule.

## Technical Solution

An aspect provides a drug complex including: a glutathione-S-transferase (GST) molecule; an antibody, an affibody molecule, or a diabody molecule, each having binding ability to human epidermal growth factor receptor 2 (HER2); a linker that links the GST with the antibody, the affibody molecule, or the diabody molecule; and 7-ethyl-10-hydroxycamptothecin bound with the GST via a glutathione (GSH) molecule.

As used in the present specification, the term "antibody" refers to, as a term known in the art, a protein molecule specifically directed against an antigenic site. For the purpose of the present specification, an antibody refers to an antibody that binds specifically to a target cell or a receptor expressed on a target cell, and for such an antibody, each gene is cloned into an expression vector according to a conventional method to obtain a protein encoded by the marker gene, and an antibody is prepared from the obtained protein by a conventional method. This also includes a partial peptide that can be produced from the aforementioned protein, and such a partial peptide of the present disclosure includes at least 7 amino acids, preferably 9 amino acids, and more preferably 12 or more amino acids. The form of the antibody of the present specification is not particularly limited, and the antibody of the present specification may include a polyclonal antibody, a monoclonal antibody, or a humanized antibody. An antigen-binding fragment that is a part of the foregoing antibodies may be also included as long as having antigen-binding property, and all immunoglobulin antibodies may be included.

As used in the present specification, the term "affibody molecule" may refer to an antibody mimic capable of binding to a specific target protein (receptor). Typically, the affibody molecule consists of 20 to 150 amino acid residues, and may consist of 2 to 10 alpha helices. More specifically, the affibody molecule may include an anti-ErbB affibody molecule (ab31889), a HER2-specific affibody molecule (ZHER2:342), an anti-EGFR affibody molecule (ZEGFR:2377), etc. In addition, the affibody molecule includes, but is not limited thereto, all affibody molecule capable of recognizing a specific receptor or a target protein of a cell. Examples of the target receptor or the target protein which may be recognized by the affibody molecule may include an amyloid beta peptide, synuclein (e.g., alpha-synuclein), an apolipoprotein (e.g., apolipoprotein A1), a complement factor (e.g., C5), a carbonic anhydrase (e.g., CAIX), an interleukin-2 receptor alpha chain (IL2RA; CD25), a CD antigen on the cell surface (e.g., CD28), or c-Jun, Factor VIII, provirogen, GP120, H-Ras, Her2, Her3, HPV16 E7, a human islet amyloid polypeptide (IAPP), immunoglobulin A (IgA), IgE, IgM, interleukins (e.g., IL-1, IL-6, IL-8, IL-17), insulin, a staphylococcal protein A domain, Raf-1, a light-oxygen-voltage-sensing domain (LOV) domain, or an RSV G protein. Information on the affibody is described in Affibody Molecules in Biotechnological and Medical Applications, Stefan Stahl et al., Trends in Biotechnology, August 2017, Vol 35, No 8, and the content of this publication is incorporated herein by reference in its entirety.

As used in the present specification, the term "linker" refers to a molecule that links two or more chemical structures. Specifically, the linker may be a polypeptide consisting of any amino acids of 1 to 400, 1 to 200, or 2 to 200. The peptide linker may include Gly, Asn and Ser residues, and may also include neutral amino acids such as Thr and Ala. The amino acid sequences suitable for the peptide linker are known in the art. In addition, to achieve appropriate separation among functional moieties or to maintain necessary inter-moiety interactions, the copy number "n" may be adjusted in consideration of optimization of the linker.

In an embodiment, the peptide linker may be a protease-resistant linker. Being resistant specifically means not being bound by a protease and/or not being cleaved by a protease and/or remaining stable upon contact with a protease, and/or retaining the original activity.

In an embodiment, the peptide linker may be a flexible linker including a G residue, an S residue, and/or a T residue. Other flexible linkers are known in the art, for example, G and S linkers including additional amino acid residues,

such as Tand A, to maintain flexibility, as well as polar amino acid residues to improve solubility. Specifically, the linker may have a general formula selected from (GpSs)n and (SpGs)n, wherein, independently, p may be an integer from 1 to 10, s may be 0 or an integer from 0 to 10, p+s may be an integer of 20 or less, and n may be an integer from 1 to 20. Examples of linker may include (GGGGS)n (SEQ ID NO:2), (SGGGG)n (SEQ ID NO: 3), (SRSSG)n (SEQ ID NO: 4), (SGSSC)n (SEQ ID NO: 5), (GKSSGSGSESKS)n (SEQ ID NO: 6), (RPPPPC)n (SEQ ID NO: 7), (SSPPPPC)n (SEQ ID NO: 8), (GSTSGSGKSSEGKG)n (SEQ ID NO: 9), (GSTSGSGKSSEGSGSTKG)n (SEQ ID NO: 10), (GSTSGSGKPGSGEGSTKG)n (SEQ ID NO: 11), or (EGKSSGSGSESKEF)n (SEQ ID NO: 12), wherein n may be an integer from 1 to 20 or an integer from 1 to 10.

Another aspect provides a polynucleotide encoding the fusion protein, wherein the fusion protein includes: the fusion protein; and the antibody, the affibody molecule, or the diabody molecule, each having binding ability to HER2.

In the present specification, the term "polynucleotide" refers to a single-stranded or double-stranded polymer of deoxyribonucleotides or ribonucleotides. The polynucleotide encompasses RNA genome sequences, DNA (gDNA and cDNA) and RNA sequences transcribed therefrom, and unless otherwise specified, includes natural polynucleotides as well as analogues thereof in which sugar or base moieties are modified. In an embodiment, the polynucleotide is a single-stranded polynucleotide.

Another aspect provides a vector including the polynucleotide.

As used in the present specification, the term "vector", which is a vector capable of expressing a target protein in an appropriate host cell, refers to a genetic construct including a regulatory element operably linked to express a gene insert. The vector according to an embodiment may include expression regulatory elements, such as a promoter, an operator, a start codon, a stop codon, a polyadenylation signal, and/or an enhancer, and the promoter of the vector may be constitutive or inducible. In addition, the vector may be an expression vector capable of stably expressing the fusion protein in a host cell. For use as the expression vector, a vector commonly used in the art to express a foreign protein in plants, animals, or microorganisms may be used. The recombinant vector may be constructed by various methods known in the art. For example, the vector may include a selectable marker for selecting a host cell including the vector, and in the case of a replicable vector, it may include an origin of replication. In addition, the vector may be self-replicable or may be introduced into a host DNA, and the vector may be selected from the group consisting of a plasmid, a lentivirus, an adenovirus, an adeno-associated virus, a retrovirus, a herpes simplex virus, and a vaccinia virus.

The vector may include a promoter operable in an animal cell, for example a mammalian cell. An appropriate promoter according to an embodiment may include promoters derived from mammalian viruses and promoters derived from genomes of mammalian cells, and examples thereof may include a cytomegalovirus (CMV) promoter, a U6 promoter an H1 promoter, a murine leukemia virus (MLV)-long terminal repeat (LTR) promoter, an adenovirus early promoter, an adenovirus late promoter, a vaccinia virus 7.5K promoter, an SV40 promoter, a tk promoter of HSV, an RSV promoter, an EF1 alpha promoter, a metallothionine promoter, a beta-actin promoter, a promoter of human IL-2 gene, a promoter of human IFN gene, a promoter of human IL-4 gene, a promoter of human lymphotoxin gene, a promoter of human GM-CSF gene, a human phosphoglycerate kinase (PGK) promoter, a mouse PGK promoter, and a survivin promoter.

In addition, in the vector, the aforementioned fusion protein may be operably linked to the promoter. As used in the present specification, the term "operably linked" refers to a functional linkage between a nucleic acid expression regulatory sequence (e.g., a promoter, a signal sequence, or an array of transcriptional regulator binding sites) and another nucleic acid sequence, wherein the expression regulatory sequence may regulate transcription and/or translation of the another nucleic acid sequence.

Another aspect provides a host cell including the fusion protein, the polynucleotide, or the vector.

The cells, e.g., eukaryotic cells, may be yeast, fungi, protozoa, plants, higher plants and insects, or amphibian cells, or mammalian cells such as CHO, HeLa, HEK293, and COS-1. For example, the cells may be cultured cells (*in vitro*), transplanted cells (graft cells), and primary cell cultures (*in vitro* and *ex vivo*) and *in vivo* cells, and may also be mammalian cells including humans cells. In addition, the organism may be a yeast, a fungus, a protozoa, a plant, a higher plant, an insect, an amphibian, or a mammal. In addition, the cells may be animal cells or plant cells.

Types of a pharmaceutically active ingredient which can be delivered to an individual by using a drug delivery carrier may include an anticancer agent, a contrast agent (dye), a hormonal agent, an anti-hormonal agent, a vitamin agent, a calcium agent, a mineral agent, a sugar agent, an organic acid agent, a protein amino acid agent, an antidote agent, an enzyme agent, a metabolic agent, a diabetes combination agent, a tissue retrieval agent, a chlorophyll agent, a pigment preparation, a tumor agent, a tumor therapeutic agent, a radiopharmaceutical product, a tissue cell diagnostic agent, a tissue cell therapeutic agent, an antibiotic preparation, an antiviral agent, a complex antibiotic agent, a chemotherapeutic agent, a vaccine, a toxin, a toxoid, an antitoxin, leptospira serum, a blood product, a biological product, an analgesics, an immunogenic molecule, an antihistamine agent, an allergic drug, a non-specific immunogen preparation, an anesthetics, a stimulant, a psychotropic agent, a small molecular compound, a nucleic acid, an aptamer, an antisense nucleic acid, an oligonucleotide, a peptide, siRNA, and micro RNA, etc.

SN-38 may be an anticancer agent, and may include a pharmaceutically acceptable salts thereof. "SN-38 (7-ethyl-10-hydroxycamptothecin)" is an active ingredient of irinotecan (also known as "CPT-11"), and exhibits an antitumor

effect by inhibiting type I DNA topoisomerase activity. The SN-38 may exhibit cytotoxic activity that is up to 1,000 times more potent than irinotecan against various cancer cells *in vitro.*

In the present disclosure, the binding of the GST and the SN-38 (7-ethyl-10-hydroxycamptothecin) may occur via GSH. That is, the SN-38 may be SN-38 to which GSH is bound. The GSH functions as a binding site of the GSH to link the SN-38 with the GST.

The SN-38 molecule may be SN-38-loaded nanoparticles or nanoparticles capable of loading the SN-38. The nanoparticles may be any nanoparticles without limitation, as long as they may be applied as a drug delivery carrier according to a known technology. Specifically, the nanoparticles may be any one selected from the group consisting of mesoporous silica nanoparticles (MSNs), gold nanoparticles, magnetic nanoparticles, nucleic acid-metal organic framework nanoparticles, and polymer nanoparticles. In addition, the nanoparticles may be nanoparticles to which GSH is bound. Accordingly, the nanoparticles may bind with the fusion protein including GST.

In an embodiment, the SN-38 molecule may be represented by Formula 1:

[Formula 1]

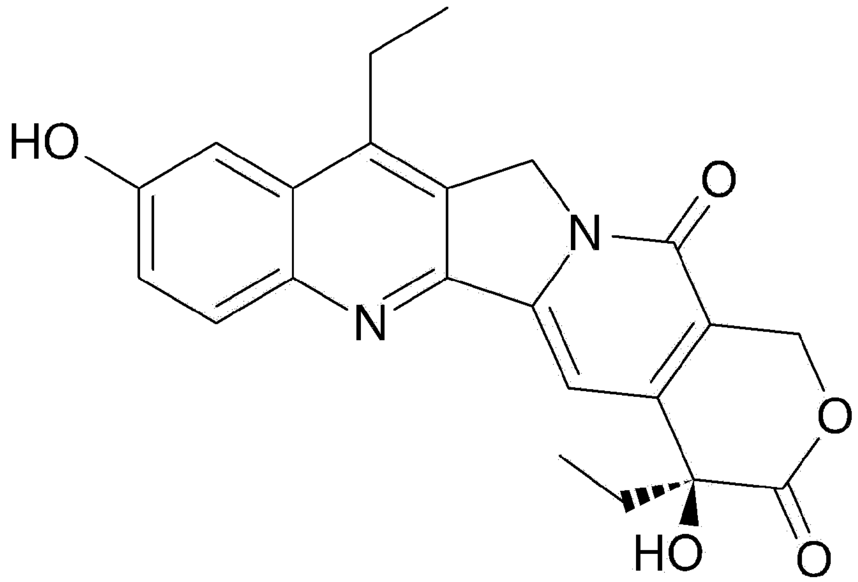

Another aspect provides a pharmaceutical composition for preventing or treating a proliferative disease, the pharmaceutical composition including: a GST molecule; an antibody, an affibody molecule, or a diabody molecule, each having binding ability to HER2; a linker that links the GST with the antibody, the affibody molecule, or the diabody molecule; and 7-ethyl-10-hydroxycamptothecin bound with the GST via a GSH molecule.

Another aspect provides a method of preventing or treating a proliferative disease, the method including administering an effective amount of a drug complex to an individual in need thereof, the drug complex including: a GST molecule; an antibody, an affibody molecule, or a diabody molecule, each having binding ability to HER2; a linker that links the GST with the antibody, the affibody molecule, or the diabody molecule; and 7-ethyl-10-hydroxycamptothecin bound with the GST via a GSH molecule.

Another aspect provides the use of a drug complex comprising a glutathione-S-transferase (GST) molecule for the manufacture of a pharmaceutical preparation for the prevention or treatment of a proliferative disease; an antibody, affibody or diabody molecule having a binding ability to HER2 (human epidermal growth factor receptor2); a linker connecting the glutathione-S-transferase and the antibody, affibody or diabody; and 7-ethyl-10-hydroxycamptothecin bound with the GST via a glutathione (GSH) molecule.

As used in the present specification, the term "proliferative disease" refers to a disease caused by abnormal expansion by enlargement of cells. The proliferative disease may be associated with the following: 1) pathological proliferation of normal resting cells; 2) pathological migration of cells from normal positions (e.g., transition of neoplastic cells); 3) pathological expression of proteolytic enzymes, such as matrix metalloproteinases (e.g., collagenase, gelatinase, and elastase); 4) pathological angiogenesis, such as those in proliferative retinopathy and tumor metastasis; or 5) evasion of host immune surveillance and neoplastic cell elimination.

The proliferative disease may include cancer (i.e., malignant neoplasm), benign neoplasm, and angiogenesis.

In an embodiment, the cancer may be blood cancer or solid cancer.

The blood cancer may be selected from the group consisting of acute myeloid leukemia, acute lymphoblastic

leukemia, chronic myelogenous leukemia, multiple myeloma, and lymphoma, but is not limited thereto.

The solid cancer may be selected from the group consisting of breast cancer, colon cancer, head and neck cancer, lung cancer, gastric cancer, brain cancer, skin cancer, colon cancer, prostate cancer, bladder cancer, kidney cancer, rectal cancer, thyroid cancer, liver cancer, cervical cancer, rectal cancer, anal cancer, urethral cancer, ovarian cancer, esophageal cancer, and pancreatic cancer, but is not limited thereto. In addition, the cancer may be at least one selected from the group consisting of gastric cancer, breast cancer, lung cancer, liver cancer, esophageal cancer, and prostate cancer, each having resistance to an anticancer agent (e.g., multidrug resistance). In addition, the cancer may be a metastatic cancer in which cancer cells that have been separated from the site where the cancer first occurred spread to other sites through blood, lymphatic vessels, etc., and proliferate.

In an embodiment, the benign neoplasm may include an adenoma, a fibroma, a hemangioma, a nodular sclerosis, and a lipoma.

As used in the present specification, the term "therapeutic agent" or "pharmaceutical composition" refers to a molecule or a compound that confers some beneficial effects upon administration to a subject. The beneficial effects may include: enablement of diagnostic determinations; amelioration of a disease, symptom, disorder, or pathological condition; reduction or prevention of the onset of a disease, symptom, disorder, or illness; and generally counteracting a disease, symptom, disorder, or pathological condition.

As used in the present specification, the term "treatment" or "treating," or "alleviating" or "ameliorating" is used interchangeably. These terms refer to methods of obtaining beneficial or desired results including, but not limited to, therapeutic benefits and/or prophylactic benefits. Therapeutic benefit means any therapeutically relevant improvement in or effect on one or more diseases, illnesses, or symptoms under treatment. For prophylactic benefits, the composition may be administered to a subject at risk of developing a particular disease, illness, or symptom, or to a subject reporting one or more physiological symptoms of a disease, even though a disease, illness or symptom has not yet manifested.

As used in the present specification, the term "effective amount" or "therapeutically effective amount" refers to an amount of an agent that is sufficient to cause a beneficial or desired result. The therapeutically effective amount may vary depending on one or more of a subject and pathological conditions being treated, the weight and age of a subject, severity of a pathological condition, administration methods, etc., which may be readily determined by a person skilled in the art. In addition, the term is applied to a dose that will provide an image for detection by any one of imaging methods described herein. A specific dose may vary depending on one or more of a particular agent chosen, a dosing regimen to be followed, whether it is administered in combination with other compounds, timing of administration, a tissue to be imaged, and a physical delivery system carrying the tissue.

The pharmaceutical composition may be parenterally administered during clinical administration, and may be used in the form of a general pharmaceutical preparation. Parenteral administration may refer to administration through an administration route, such as rectal, intravenous, peritoneal, intramuscular, intraarterial, transdermal, nasal, inhalation, ocular, or subcutaneous routes, other than an oral route. When the pharmaceutical composition of the present disclosure is used as a medical product, at least one active ingredient that exhibits the same or similar function may be further included.

When formulating the pharmaceutical composition, it is prepared by using a commonly used diluent or excipient, such as a filler, an extender, a binder, a wetting agent, a disintegrating agent, a surfactant, etc. Formulations for parenteral administration may include sterile aqueous solutions, non-aqueous solvents, suspensions, emulsions, lyophilized preparations, and suppositories. As the non-aqueous solvent and a suspension solvent, propylene glycol, polyethylene glycol, vegetable oil, such as olive oil, injectable ester, such as ethyl oleate, etc. may be used. As a base for the suppository, Witepsol, Macrogol, Tween 61, cacao butter, laurin butter, glycerogelatin, etc. may be used.

In addition, the pharmaceutical composition may be used by mixing with various pharmaceutically acceptable carriers, such as physiological saline or organic solvents. To increase stability or absorption, carbohydrates, such as glucose, sucrose, or dextran, antioxidants, such as ascorbic acid or glutathione, chelating agents, low-molecular proteins, or other stabilizers may be used as pharmaceutical agents.

The effective dose of the pharmaceutical composition may be 0.01 to 100 mg/kg, preferably 0.1 to 10 mg/kg, and may be administered once to three times a day.

As used in the present specification, the terms "subject," "individual," and "patient" are used interchangeably herein to designate vertebrates, preferably mammals, and more preferably, humans. The mammals include, but are not limited to, murines, monkeys, humans, farm animals, sport animals, and pets. Tissues and cells of a biological entity obtained *in vivo* or cultured *in vitro,* and progeny thereof are also included.

Still another aspect provides a protein corona shield nanoparticle (PCSN) including the following components: a) a nanoparticle capable of loading a drug; b) a fusion protein including GST which is bound to the surface of the nanoparticle.

In addition, the present disclosure provides a nanoparticle drug delivery carrier or a drug complex, each having a protein corona shield, wherein a drug is loaded in the nanoparticle.

In addition, the present disclosure provides a method of preparing a nanoparticle drug delivery carrier or a drug

complex, each having a protein corona shield, the method including the following i) to iii):
i) binding a linker (e.g., GSH) to the surface of a nanoparticle; ii) loading a drug inside or on the surface of the nanoparticle bound with the linker in i); and iii) coating the surface of the drug-loaded nanoparticle in ii) with GST fusion protein to form a protein corona shield (PCS).

In the PCSNs, the PCS is first formed by pre-coating the surface with the fusion protein, and therefore, a corona layer surrounded by serum proteins is not formed in the body environment. Accordingly, the nanoparticles may avoid immune responses by macrophages, thereby having a significant stealth effect. Therefore, the PCSNs may not only sustain *in vivo* retention time but also improve targeting ability to a target cell to be effectively delivered to the target cell, thereby being usefully applied as a targeted therapeutic agent.

## Advantageous Effects

A fusion protein according to an aspect and a drug delivery carrier or a drug complex, each including the same, may not only sustain *in vivo* residence time but also improve targeting ability to a target cell to be effectively delivered to the target cell, thereby being usefully applied to a targeted therapeutic agent.

## Description of Drawings

FIG. 1 is a graph showing SDS-PAGE analysis results of GST-HER2 Afb.
FIG. 2 is an image of confirming expression levels of HER2-positive breast cancer cell line SK-BR3, gastric cancer cell line NCI-N87, and HER2-negative breast cancer cell line MDA-MB231.
FIG. 3 is a graph showing cell viability of HER2-positive breast cancer cell line SK-BR3, gastric cancer cell lines NCI-N87 and MKN45, and HER2-negative breast cancer cell line MDA-MB231, after treatment with KMD111.
FIG. 4 is a graph showing changes in body weight according to doses of KMD111 in a mouse animal model transplanted with gastric cancer cell line MKN45.
FIG. 5 is a graph showing results of confirming antitumor effects according to doses of KMD111 in a mouse animal model transplanted with gastric cancer cell line MKN45.

## Mode for Invention

Hereinafter, preferable examples are presented to help understanding of the present disclosure. However, examples below are only presented only for easier understanding of the present disclosure, and the contents of the present disclosure are not limited by these examples. Since examples may apply various modifications, examples are not limited to those described herein and may be implemented in various forms.

### Example 1. Preparation of fusion protein of affibody and glutathione-S-transferase (GST)

In the present disclosure, by forming a protein corona shield on the surface of nanoparticles that are to be used as drug delivery carriers, a drug delivery carrier having improved stability and targeting ability even in *in vivo* environment was prepared. In this regard, a fusion protein capable of constituting the protein corona shield was first prepared. The fusion protein was expressed in such a form that GST was bound with affibody (Afb) to be able to bind specifically to receptors on the surface of cancer cells.

Specifically, in the present disclosure, HER2 Afb binding specifically to HER2 was used as the Afb. In GST protein, human GSTA1 (P08263) having SEQ ID NO: 13 was introduced, a linker sequence SGGGSGGGSGGGSGGGSGGGSGGG (SEQ ID NO: 1) was ligated to the end of the C-terminal of GST, and then, HER2-affibody (ZHER2:342) having SEQ ID NO: 14 was introduced. Based on the amino acid sequence of the HER2-affibody-GST fusion protein having SEQ ID NO: 15, codon optimization to induce expression in E. *coli* was performed, and the encoded gene (SEQ ID NO: 16) was synthesized through Invitrogen GeneArt gene synthesis (ThermoFisher) and was inserted into a protein expression plasmid, pET151/D-TOPO.

The constructed plasmid was inserted (transformation) into an E. *Coli* BL21 (DE3) strain, and then cultured. The culture solution was treated with IPTG to induce expression of the fusion protein, cultured at 30 °C for 16 hours, and centrifuged to obtain cells. The obtained cultured cells were suspended in 50 mM PBS and disrupted by using a high-pressure cell disruptor. By centrifugation after the disruption, a supernatant containing the fusion protein was recovered, and the HER2-affibody-GST was purified by NI-NTA affinity chromatography using FPLC equipment. The purified protein was subjected to SDS-PAGE analysis to analyze the protein purity and molecular weight.

FIG. 1 is a graph showing the SDS-PAGE analysis results of the HER2-affibody-GST.

Table 1 shows quantitative results of the protein purity obtained by quantifying the SDS-PAGE analysis of the HER2-affibody-GST.

[Table 1]

| Band No. | Mol. Wt.(KD a) | Relative Front | Adj. Volume(Int) | Volume(Int) | Abs. Quant. | Rel. Quant. | Band% | Lane % |
|---|---|---|---|---|---|---|---|---|
| 1 | N/A | 0.275 | 309,225 | 27,068,370 | N/A | N/A | 0.4 | 0.3 |
| 2 | N/A | 0.537 | 87,572,730 | 176,874,915 | N/A | N/A | 99.5 | 74.0 |
| 3 | N/A | 0.646 | 173,040 | 34,072,605 | N/A | N/A | 0.2 | 0.1 |
| Band Detection | | | Automatically detected bands with sensitivity: High | | | | | |
| Lane Background | | | Lane background subtracted with disk size: 0.1 | | | | | |
| Lane Width | 7.17 mm | | | | | | | |

As shown in FIG. 1 and Table 1, the HER2-affibody-GST protein having a purity of 99.5% was purified.

**Example 2. Preparation of drug delivery carrier having protein corona shield (PCS)**

**<2-1> Preparation of mesoporous silica nanoparticles**

As the basic structure of the drug delivery carrier, mesoporous silica nanoparticles (MSNs) were prepared.

First, to prepare MSNs with a diameter of 50 nm or less, 1.53 g of CTAB and 0.3 g of tetraethylammonium hydroxide (TEAH) solution were added to 100 g of deionized water and dissolved by stirring at 80 °C for 1 hour. When CTAB and TEAH were completely dissolved, 14.45 g of TEOS was added and further stirred at 800 rpm for 2 hours. When the stirred solution appeared in a white solid phase, it was filtered through a vacuum filter, washed with deionized water, and dried in air at 70 °C to obtain a dried product. The dried product thus obtained was homogenized by using an agate mortar and then calcined at 550 °C for 5 hours to finally obtain MSNs.

**<2-2> Preparation of nanoparticles of which surface was bound with GSH**

As a process of preparing the PCSNs of the present disclosure, nanoparticles (GSH-modified particles, MMSNs), of which surface was bound with glutathione (GSH) by thiol-ene click chemistry, were prepared.

100 mg of the MSNs prepared in Example <2-1> and 1 ml of 3-(trimethoxysilyl) propyl acrylate were mixed in 18 ml of toluene. The mixed solution was allowed to react by stirring at 60 °C for 24 hours. After the reaction, the reacted MSNs were washed with ethanol and deionized water, and then added to 16 ml of DMF to prepare MSNs. GSH for shield formation was prepared by dissolving 100 mg of GSH in 2 ml of deionized water. Then, the prepared MSNs-containing solution and GSH aqueous solution were mixed, and 40 μl of pyridine was added thereto and stirred by vortexing. After the stirring, the mixed solution was left at room temperature for 72 hours to induce a reaction. After termination of the reaction, the nanoparticles were washed with ethanol three times, and vacuum-dried at room temperature to finally obtain nanoparticles (MMSN) of which surface was bound with GSH.

**<2-3> Preparation of drug-loaded nanoparticles**

To prepare protein corona shield nanoparticles (PCSNs) for use as a drug delivery carrier, nanoparticles with a GST-Afb protein corona shield were created in the present invention. MSNs are used as a carrier capable of loading a substance via a chemical functional group inside of or on the surface of the MSNs. In this regard, in the present disclosure, MMSNs were prepared by binding GSH to the surface of the MSNs, and PCSNs were prepared by forming a protein corona shield bound via GST.

Specifically, to load a drug on the GSH-MSN prepared in Example <2-2>, 5 mg of the GSH-MSN was dispersed in 1 ml of DMSO, and a DMSO solution (10 mg/ml) containing SN-38 dissolved therein was slowly added thereto, followed by stirring at room temperature for 12 hours. After stirring, SN-38 loaded GSH-SN-38 MSN was recovered by centrifugation, washed with D.I water three times, and then vacuum-dried. The drug loading ratio was calculated by the following equation.

Drug loading capacity (%) = Mass of drug in GSH-MSN / Mass of GSH- MSN x 100 [Equation 1]

Next, to prepare PCSNs, 1 mg of the HER2-affibody-GST protein was prepared and dissolved in 2 ml of PBS to prepare a protein solution. After dispersing 1 mg of the SN-38-loaded GSH-SN-38 MSN in 3 ml of PBS, the prepared protein solution was slowly added dropwise and mixed while stirring at 4 °C, mixed, and stirred for additional 2 hours. After the stirring, centrifugation was performed to remove unbound proteins, followed by washing with PBS three times. Finally, PCSNs (KMD111), which are the HER2-affibody-GST-SN-38 MSNs, were obtained and stored in PBS for use in the following efficacy evaluation experiments.

**Experimental Example 1. Confirmation of HER2 expression level in each cell**

Before confirming cytotoxicity of the KMD111 prepared in Example 2, the HER2 expression level in each cell line was first confirmed. An HER2 receptor is a cell membrane receptor belonging to the ERBB/HER growth factor superfamily, and an HER2 gene is a well-known proto-oncogene. A KMD111 candidate is a targeted drug delivery system that conjugates an affibody specifically targeting HER2 to a nanoparticle loaded with SN-38, which is topoisomerase that inhibits DNA replication, and the higher the HER2 expression level is, the greater the apoptosis effect is expected.

Cells used in the experiment were human breast cancer cell lines SKBR3 and MDA-MB231 and human gastric cancer cell lines NCI-N87 and MKN4 purchased from the Korean Cell Line Bank. An RPMI-1640 medium (Thermo fisher scientific, USA) supplemented with 10 % fetal bovine serum (FBS, Hyclone, Logan, UT, USA) and 1 % stereptomycin-peniciline (Invitrogen, Carlsbad, CA, USA) was used and cultured in a 5 % $CO_2$ incubator at 37 °C. Cells used in each experiment were subcultured when the cells reached a confluency of about 80 % to 90 %.

Specifically, the HER2 expression levels of the cell lines used in the experiment were confirmed by western blotting. When the cell lines grew to a density of about 80 % to 90 % in a culture dish, the cells were disrupted with a RIPA buffer containing protease/phosphatase inhibitors and centrifuged to obtain a cell lysate. 10 ug of protein per well was loaded onto Nu-PAGE 4 to 12 % bis-tris gel, developed, and transferred to a PVDF membrane. The PVDF membrane to which the proteins were transferred was blocked, and the signals were obtained by reacting in the order of primary antibodies, secondary antibodies, and HRP substrates. These results are shown in FIG. 2.

As shown in FIG. 2, it was confirmed that the HER2 expression level was very low in the HER2-negative breast cancer cell line MDA-MB231, whereas the HER2-positive breast cancer cell line SKBR3 and the gastric cancer cell line NCI-N87 expressed a high level of HER2.

Experimental Example 2. Confirmation of cytotoxicity of HER2-affibody-GST-SN-38 (KMD111) on target cells

To confirm the drug delivery ability of the KMD111 prepared in Example 2 to target cells, cytotoxicity against target cells was confirmed.

Specifically, MDA-MB231 cells of a HER2-negative human breast cancer cell line as a normal control group, SK-BR3 cells of a breast cancer cell line that overexpresses a receptor recognized by HER2 Afb, and gastric cancer cell lines NCI-N87 and MKN-45 were seeded in a 96-well plate at a density of $1\times10^4$ cells/well, cultured for 24 hours, treated with KMD111 at different concentrations, and then, cultured for 72 hours. On the basis of the concentration of SN-38, the cells were treated at concentrations of 3.1, 6.2, 12.5, 25, 50, 100, and 200 ng/mL, respectively. After treated 96 wells with 10 $\mu$L of cck-8 (Dojindo, Japan) on the cultured plate, the cells were cultured for 1 hour. Here, a highly water-soluble tetrazolium salt, WST-8, is reduced by the dehydrogenase activity of the cells, and produces yellow formazan dyes that dissolve in a tissue culture medium. In this regard, the measured absorbance value is proportional to the number of viable cells. The absorbance at 450 nm was measured by using a microplate reader (Multiskan SkyHigh Microplate Spectrophotometer, Thermo fisher scientific, USA) to indicate cell viability compared to the control group, and the results are shown in FIG. 3.

As shown in FIG. 3, the KMD111 showed a cytotoxicity of less than 30 % against the MDA-MB231 cells that do not express HER2, and an apoptosis efficacy of 80 % or more in the SKBR3 cells and an apoptosis efficacy of 70 % or more in the NCI-N87 of a gastric cancer cell line were confirmed. Accordingly, since the KMD111 may exhibit the apoptosis effect specifically against the HER2-positive cancer cells, a significant anticancer effect may be exhibited when the KMD111 is used as a drug delivery carrier.

**Experimental Example 3. *In vivo* efficacy evaluation using HER2-affibody-GST-SN-38 MSN (KMD111)**

To confirm the anticancer effect of the KMD111 against target tumor cells, tests were performed in an *in vivo* environment using a mouse animal model transplanted with a human gastric cancer cell line MKN45.

Specifically, for the anticancer efficacy test, 6-week-old NIG mice (NOD/SCID, GH Bio Co., Ltd.) were purchased and bred in an environment of free access to feed and water, acclimated for more than 10 days, and then transplanted with tumor cells. As the tumor cells, HER2-positive gastric cancer MKN45 cells were subcutaneously administered at a dose of $5\times10^6$ cells/100 $\mu$L onto a fixed site on the left flank. After 1 week, a tumor was measured until the start of test substance

administration, and individuals having tumors reached a size of about 150 $mm^3$ were selected and randomly distributed to each test group. Then, administration began.

The test group was divided into a PBS-administered control group and KMD111-administered groups at 3 mg/kg, 6 mg/kg, and 12 mg/kg based on SN-38, having three mice per group. Intravenous injection (IV) was performed once every three days for 21 days, a total of eight times. Then, the tumor size and body weight were measured from Day 11 after the cancer cell transplantation (Day 0) to the end date (for 32 days). The tumor size, relative tumor volume (RTV), and tumor growth inhibition % (TGI %) were calculated according to the following equations, by measuring a long axis length and a short axis length of the tumor.

Equation 2]

Tumor size ($mm^3$) = (length x $width^2$)/2, (L: long axis, W: short axis)

[Equation 3]

RTV = (tumor size on final day)/(tumor size on initial day)

TGI % = [1 - (RTV of the treated group)/(RTV of the control group)] x 100 (%) [Equation 4]

The results of body weight checked from each test animal are shown in FIG. 4, and the results of analyzing the tumor size are shown in FIG. 5.

As shown in FIG. 4, there was no weight gain according to age in all groups, and weight loss was observed in the KMD111-administered group. However, no special clinical symptoms were observed in the same group, and the animals were in good condition.

As shown in FIG. 5, when tumor size was analyzed, there was a significant reduction in tumor size in a concentration-dependent manner in the KMD111-administered group compared to the PBS-administered group. Compared to the PBS-administered control group, the tumor growth inhibition % (TGI %) of the KMD111-administered groups, i.e., KMD111-3 mg/kg, KMD111-6 mg/kg, and KMD111-12 mg/kg administration groups, were 79.5 %, 83.5 %, and 92.5%, respectively, confirming that tumor progression was inhibited. In other words, the KMD111 inhibited tumor growth at all concentrations in the xenograft mouse model transplanted with MKN45 cell line, indicating that the KMD111 has a distinct anticancer effect against MKN45 tumor.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

**1.** A drug complex comprising: a glutathione-S-transferase (GST) molecule;

an antibody, an affibody molecule, or a diabody molecule, each having binding ability to human epidermal growth factor receptor 2 (HER2);
a linker that links the GST with the antibody, the affibody molecule, or the diabody; and
a 7-ethyl-10-hydroxycamptothecin molecule bound with the GST via a glutathione (GSH) molecule.

**2.** The drug complex of claim 1, wherein the 7-ethyl-10-hydroxycamptothecin molecule is represented by Formula 1:

[Formula 1]

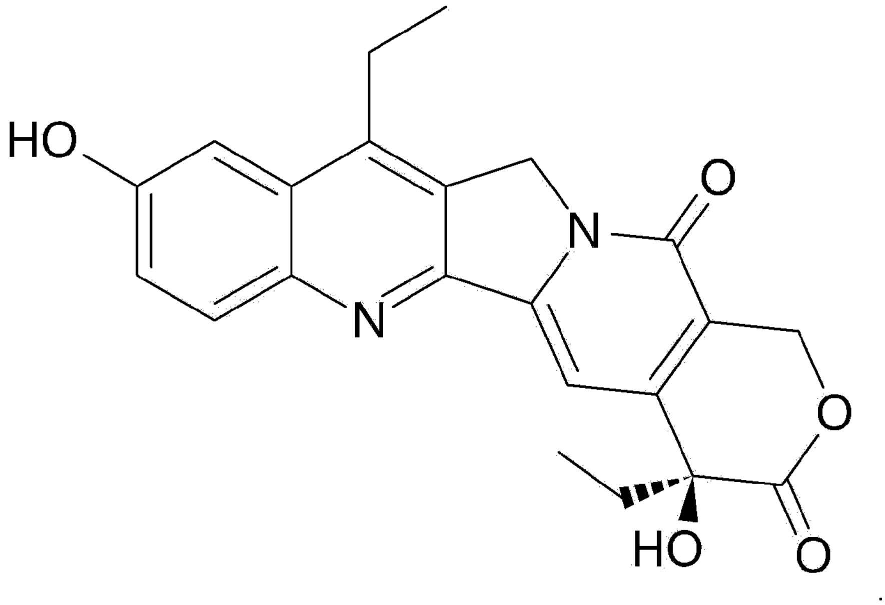

3. The drug complex of claim 1, wherein the affibody is a HER2-specific affibody having a SEQ ID NO: 14.

4. The drug complex of claim 1, wherein the linker does not bind to a protease.

5. The drug complex of claim 1, wherein the 7-ethyl-10-hydroxycamptothecin molecule is loaded on a nanoparticle.

6. The drug complex of claim 5, wherein the nanoparticle is any one selected from the group consisting of a mesoporous silica nanoparticles (MSN), a gold nanoparticle, a magnetic nanoparticle, a nucleic acid-metal organic framework nanoparticle, and a polymer nanoparticle.

7. A pharmaceutical composition for preventing or treating a proliferative disease, the pharmaceutical composition comprising a drug complex as an active ingredient, wherein the drug complex comprises: a glutathione-S-transferase (GST) molecule;

an antibody, an affibody molecule, or a diabody molecule, each having binding ability to human epidermal growth factor receptor 2 (HER2);
a linker that links the GST with the antibody, the affibody molecule, or the diabody; and
a 7-ethyl-10-hydroxycamptothecin molecule bound with the GST via a glutathione (GSH) molecule.

8. The pharmaceutical composition of claim 7, wherein the 7-ethyl-10-hydroxycamptothecin molecule is represented by Formula 1:

[Formula 1]

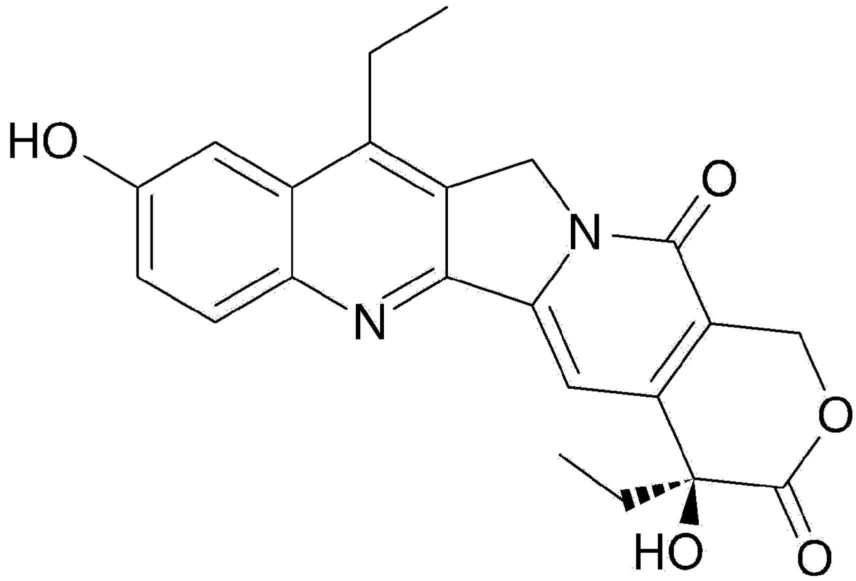

**9.** The pharmaceutical composition of claim 7, wherein the proliferative disease is any one selected from the group consisting of cancer, benign neoplasm, and angiogenesis.

**10.** The pharmaceutical composition of claim 9, wherein the cancer is any one selected from the group consisting of breast cancer, colon cancer, head and neck cancer, lung cancer, gastric cancer, brain cancer, skin cancer, colon cancer, prostate cancer, bladder cancer, kidney cancer, rectal cancer, thyroid cancer, liver cancer, cervical cancer, rectal cancer, anal cancer, urethral cancer, ovarian cancer, esophageal cancer, and pancreatic cancer.

**11.** The pharmaceutical composition of claim 7, wherein the 7-ethyl-10-hydroxycamptothecin molecule is loaded on a nanoparticle.

**12.** The pharmaceutical composition of claim 11, wherein the nanoparticle is any one selected from the group consisting of a mesoporous silica nanoparticles (MSN), a gold nanoparticle, a magnetic nanoparticle, a nucleic acid-metal organic framework nanoparticle, and a polymer nanoparticle.

**13.** A method of preventing or treating a proliferative disease, the method comprising administering an effective amount of the complex of claim 1 to a subject in need thereof.

**14.** Use of the complex of claim 1 for the manufacture of a pharmaceutical preparation for preventing or treating a proliferative disease.

FIG. 1

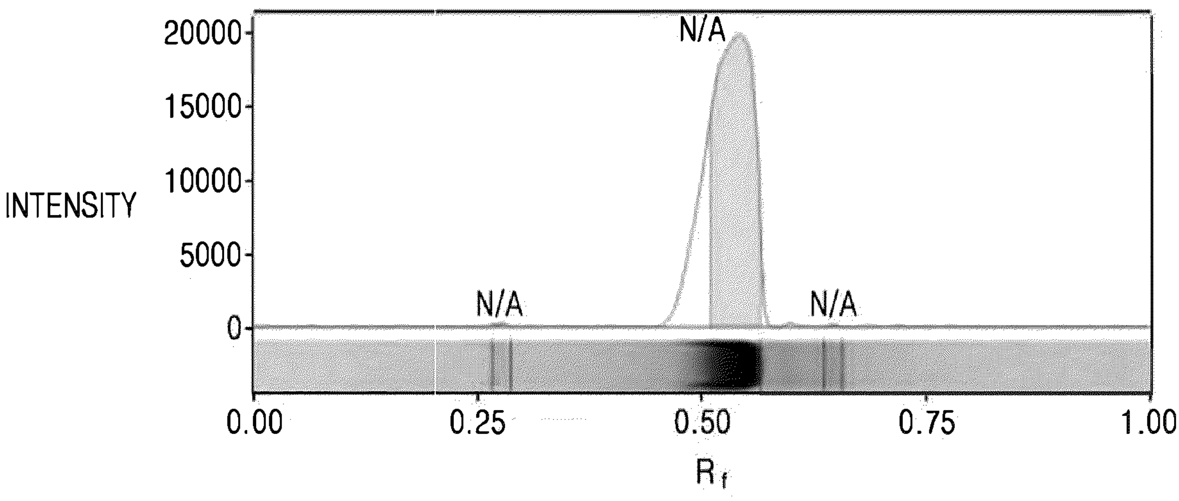
INTENSITY
20000
15000
10000
5000
0
N/A
N/A
N/A
0.00
0.25
0.50
0.75
1.00
$R_f$

FIG. 2

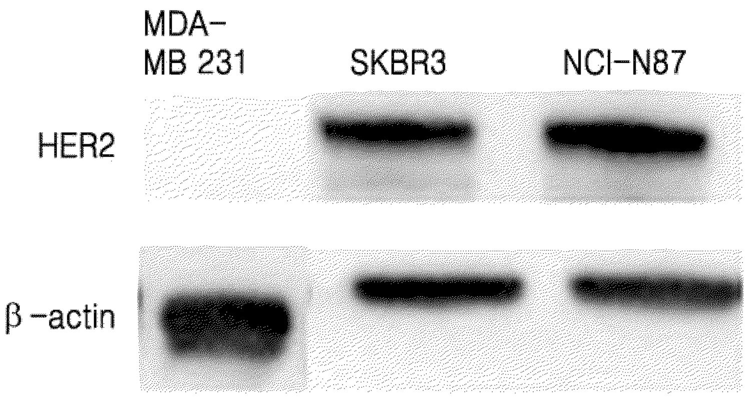

FIG. 3

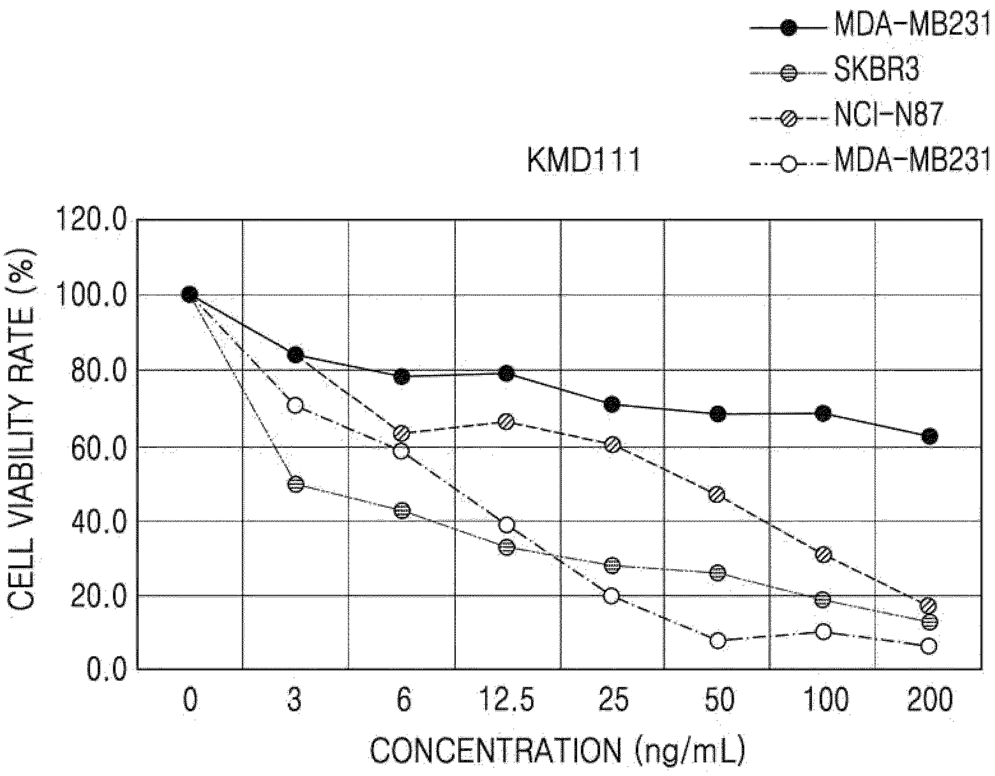
MDA-MB231
SKBR3
NCI-N87
KMD111
MDA-MB231
120.0
100.0
80.0
60.0
40.0
20.0
0.0
CELL VIABILITY RATE (%)
0
3
6
12.5
25
50
100
200
CONCENTRATION (ng/mL)

FIG. 4

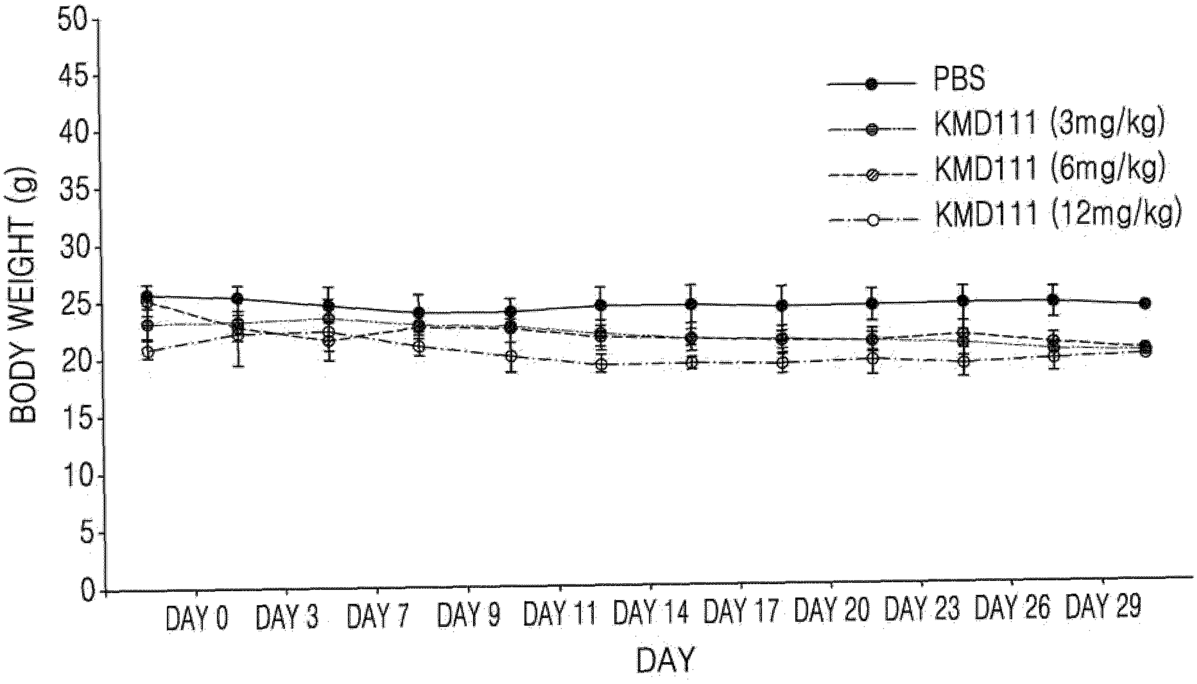
PBS
KMD111 (3mg/kg)
KMD111 (6mg/kg)
KMD111 (12mg/kg)
BODY WEIGHT (g)
50
45
40
35
30
25
20
15
10
5
0
DAY 0
DAY 3
DAY 7
DAY 9
DAY 11
DAY 14
DAY 17
DAY 20
DAY 23
DAY 26
DAY 29
DAY

FIG. 5

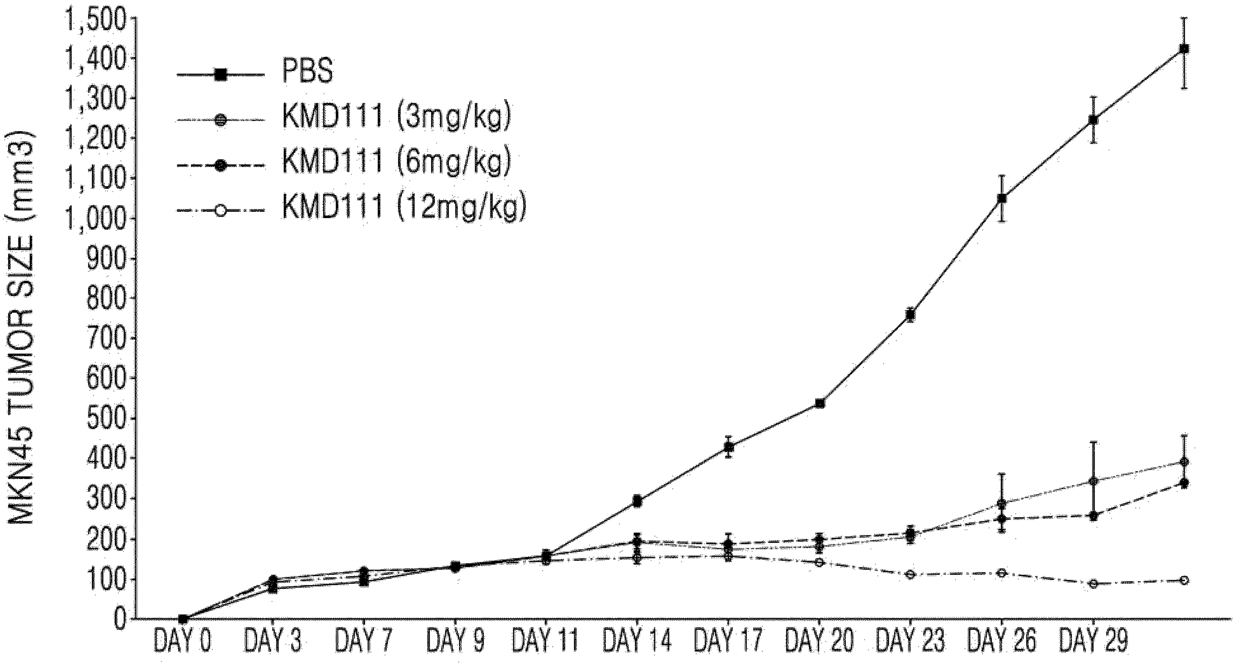
MKN45 TUMOR SIZE (mm3)
1,500
1,400
1,300
1,200
1,100
1,000
900
800
700
600
500
400
300
200
100
0
PBS
KMD111 (3mg/kg)
KMD111 (6mg/kg)
KMD111 (12mg/kg)
DAY 0
DAY 3
DAY 7
DAY 9
DAY 11
DAY 14
DAY 17
DAY 20
DAY 23
DAY 26
DAY 29

# INTERNATIONAL SEARCH REPORT

International application No.
**PCT/KR2023/006685**

## A. CLASSIFICATION OF SUBJECT MATTER

**A61K 47/64**(2017.01)i; **A61K 47/68**(2017.01)i; **A61K 31/437**(2006.01)i; **A61P 35/00**(2006.01)i

According to International Patent Classification (IPC) or to both national classification and IPC

## B. FIELDS SEARCHED

Minimum documentation searched (classification system followed by classification symbols)

A61K 47/64(2017.01); A61K 47/69(2017.01); C07K 14/31(2006.01); C07K 14/705(2006.01); C07K 16/40(2006.01); C07K 16/46(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 글루타치온-S-전이효소 (glutathione-S-transferase), 항체 (antibody), 캄토테신 유도체 (camptothecin derivatives)

## C. DOCUMENTS CONSIDERED TO BE RELEVANT

| Category* | Citation of document, with indication, where appropriate, of the relevant passages | Relevant to claim No. |
|---|---|---|
| X<br>Y | KR 10-2020-0047481 A (UNIST(ULSAN NATIONAL INSTITUTE OF SCIENCE AND TECHNOLOGY)) 07 May 2020 (2020-05-07)<br>See abstract; claims 1-6; paragraph [0045]; and figures 16a, 16b and 17a. | 1,2,4-12,14<br>3 |
| Y | US 7993650 B2 (CARLSSON, J. et al.) 09 August 2011 (2011-08-09)<br>See figure 1A. | 3 |
| A | KR 10-2017-0028637 A (UNIST(ULSAN NATIONAL INSTITUTE OF SCIENCE AND TECHNOLOGY)) 14 March 2017 (2017-03-14)<br>See entire document. | 1-12,14 |
| A | JOHNSTON, M. C. et al. Antibody conjugated nanoparticles as a novel form of antibody drug conjugate chemotherapy. Drug discovery today: Technologies. 2018, vol. 30, pp. 63-69.<br>See entire document. | 1-12,14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 August 2023** | **17 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/006685**

**C. DOCUMENTS CONSIDERED TO BE RELEVANT**

| Category* | Citation of document, with indication, where appropriate, of the relevant passages | Relevant to claim No. |
|---|---|---|
| A | ADAMS, D. J. et al. Camptothecin analogues with enhanced antitumor activity at acidic pH. Cancer chemotherapy and pharmacology. 2000, vol. 46, pp. 263-271. See entire document. | 1-12,14 |

Form PCT/ISA/210 (second sheet) (July 2022)

**INTERNATIONAL SEARCH REPORT**

International application No.
**PCT/KR2023/006685**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/006685**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

Claim 13 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/KR2023/006685**

| Patent document cited in search report | Publication date (day/month/year) | Patent family member(s) | Publication date (day/month/year) |
|---|---|---|---|
| KR 10-2020-0047481 A | 07 May 2020 | EP 3718540 A1 | 07 October 2020 |
| | | KR 10-2019-0062320 A | 05 June 2019 |
| | | KR 10-2020-0047482 A | 07 May 2020 |
| | | KR 10-2112269 B1 | 19 May 2020 |
| | | KR 10-2114445 B1 | 21 May 2020 |
| | | US 2021-0000971 A1 | 07 January 2021 |
| | | WO 2019-107896 A1 | 06 June 2019 |
| US 7993650 B2 | 09 August 2011 | AT 416190 T | 15 December 2008 |
| | | AU 2004-253835 A1 | 13 January 2005 |
| | | AU 2004-253835 B2 | 29 January 2009 |
| | | CA 2531238 A1 | 13 January 2005 |
| | | CA 2531238 C | 24 February 2015 |
| | | CN 1816563 A | 09 August 2006 |
| | | CN 1816563 B | 09 February 2011 |
| | | DK 1641818 T3 | 16 March 2009 |
| | | EP 1641818 A1 | 05 April 2006 |
| | | EP 1641818 B1 | 03 December 2008 |
| | | ES 2319426 T3 | 07 May 2009 |
| | | HK 1092481 A1 | 09 February 2007 |
| | | JP 2007-537700 A | 27 December 2007 |
| | | JP 2011-229531 A | 17 November 2011 |
| | | JP 4871126 B2 | 08 February 2012 |
| | | JP 5689022 B2 | 25 March 2015 |
| | | US 2010-0048868 A1 | 25 February 2010 |
| | | WO 2005-003156 A1 | 13 January 2005 |
| KR 10-2017-0028637 A | 14 March 2017 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STEFAN STAHL et al.** Affibody Molecules in Biotechnological and Medical Applications. *Trends in Biotechnology*, August 2017, vol. 35 (8)